# EUROPEAN PATENT APPLICATION

(11) **EP 3 018 622 A1**
(43) Date of publication of application: **11.05.2016**
(21) Application number: 15192220.0
(22) Date of filing: 29.10.2015
(51) Int. Cl.: G06Q 30/02, G06Q 50/22, G06Q 10/08

(54) **SYSTEM AND METHOD FOR MOTIVATING CONSUMERS TO MAKE HEALTHY PURCHASE DECISIONS**

(30) Priority: 30.10.2014 US 201462072475 P
(71) Applicant: Gonen, Ron, New York, NY 10023 (US)
(72) Inventor: Gonen, Ron, New York, NY 10023 (US)
(74) Representative: Casey, Alan Michael

(57) **Abstract**

A system and method for executing a rewards and loyalty program are disclosed. The system receives a food order from a user and determines a food density of food items in the food order. Further, the system retrieves a user profile and user preferences associated with the user. Thereafter, a healthy food item is recommended to the user along with reward points associated with the healthy food item based on the user profile and the user preferences.

## Description

### CROSS-REFERENCED TO RELATED APPLICATIONS

This application claims the benefit of United States Patent Application Serial No. 62/ 072,475 filed October 30, 2014, entitled "SYSTEM AND METHOD FOR MOTIVATING CONSUMERS TO MAKE HEALTHY PURCHASE DECISIONS" which is incorporated herein by reference in its entirety.

### BACKGROUND

### Field

Embodiments of the presently claimed invention generally relate to a system and method for motivating consumers to make healthier buying decisions and in particular to a system and method for motivating consumers to buy healthier food and drink at restaurants and food stores.

### Related Art

A significant number of individuals around the world are overweight and, in some instances, classified as obese. It is also well known that being overweight can be the cause, directly or indirectly, of many health ailments and diseases such as high blood pressure and high cholesterol. Being overweight is costly not only to the overweight individual but to their families, healthcare companies, and society in general. In addition, overweight individuals have been shown to underperform at routine tasks in both work and school.

More and more people are eating at restaurants and fast food establishments and making unhealthy food choices. Generally, people find losing weight and/ or following a healthier life style a difficult task. Incentives may generally be provided to people to encourage their participation and compliance to healthier diets, thereby greatly increasing their chances of meeting their health and life style goals. However, known attempts to provide incentives lack consistency, compliance, continuity, and comprehensiveness. For example, healthcare companies offer discounts on healthcare premiums or reward checks if a person attends a gym on a regular basis, logs their attendance and proves to the insurance company that a fitness plan is being followed for an extended period of time with defined results. However, if the person loses motivation to attend a gym on a regular basis, the insurance company will likely withdraw the incentive(s).

Therefore, there is a need for a comprehensive, incentive-based reward and loyalty system, and method for motivating people to make healthier buying decisions at restaurants and supermarkets.

### SUMMARY

Embodiments of the presently claimed invention generally relate to a system and method for motivating consumers to make healthier food and drink decisions. In an embodiment, there is provided a system that includes a computer readable memory comprising computer readable instructions, the memory communicatively coupled to a processor. The processor is configured to execute the computer readable instructions. The system includes a service module instructing the processor to receive a food order from a user and to determine a food density of food items in the food order. The system further includes a profile module instructing the processor to retrieve a user profile and user preferences associated with the user. The service module further instructs the processor to recommend a healthier food item along with reward points associated with the healthier food item based on the user profile and the user preferences when the determined food density is greater than or equal to a threshold.

In an embodiment, there is provided a computer implemented method for executing a rewards and loyalty program. The method includes a computer readable memory comprising computer readable instructions, the memory communicatively coupled to a processor. The processor configured to execute the computer readable instructions to receive a food order from a user. The method includes determining a food density of food items in the food order. The method further includes retrieving a user profile and user preferences associated with the user. The method further includes recommending a healthier food item along with reward points associated with the healthier food item based on the user profile and the user preferences when the determined food density is greater than or equal to a threshold.

### BRIEF DESCRIPTION OF THE DRAWINGS

So the manner in which the above recited features of the presently claimed invention may be understood in detail, a more particular description of the presently claimed invention, briefly summarized above, may be had by reference to embodiments of the present invention, several of which are illustrated in the appended drawings.

Figures in the appended drawings, like the detailed description, are examples. As such, the Figures and the detailed description are not to be considered limiting, and other equally effective examples are possible and likely. Furthermore, like reference numerals in the Figures indicate like elements, and wherein:
FIG. 1 illustrates a rewards system for executing a rewards and loyalty program coupled to a user device and other systems via a network, according to an embodiment of the present invention;
FIG. 2 illustrates the architecture of the rewards system, according to an embodiment of the present invention;
FIG. 3 illustrates the architecture of the user device, according to an embodiment of the present invention;
FIG 4 illustrates snap view of a user interface of the user device, according to an embodiment of the present invention;
FIG 5 illustrates another snap view of the user interface of the user device, according to an embodiment of the present invention;
FIG 6 illustrates another snap view of the user interface of the user device, according to an embodiment of the present invention;
FIG 7 illustrates another snap view of the user interface of the user device, according to an embodiment of the present invention;
FIG 8 illustrates another snap view of the user interface of the user device, according to an embodiment of the present invention;
FIGs 9A-9D illustrate yet other snap views of the user interface of the user device, according to an embodiment of the present invention;
FIG. 10 illustrates an exemplary method for creating a user profile at the rewards system, according to an embodiment of the present invention;
FIG. 11 illustrates an exemplary method for fulfilling a food order at the rewards system, according to an embodiment of the present invention; and
FIG. 12 illustrates an exemplary method for redeeming reward points by the user, according to an embodiment of the present invention;

The headings used herein are for organizational purposes only and are not meant to be used to limit the scope of the description or the claims. As used throughout this application, the word "may" is used in a permissive sense (*i.e.,* meaning having the potential to), rather than the mandatory sense (*i.e.,* meaning must). Similarly, the words "include," "including," and "includes" mean including but not limited to.

### DETAILED DESCRIPTION

In the following detailed description, numerous specific details are set forth in order to provide a thorough understanding of embodiments or other examples described herein. In some instances, well-known methods, procedures, components and circuits have not been described in detail, so as to not obscure the following description.

Further, the examples disclosed are for exemplary purposes only and other examples may be employed in lieu of, or in combination with, the examples disclosed. It should also be noted the examples presented herein should not be construed as limiting of the scope of embodiments of the present disclosure, as other equally effective examples are possible and likely.

The phrases "at least one", "one or more", and "and/ or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/ or C" means A alone, B alone, C alone, A and B together, A and C together, B and C together, or A, B and C together.

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The term "automatic" and variations thereof, as used herein, refers to any process or operation done without material human input when the process or operation is performed. However, a process or operation can be automatic, even though performance of the process or operation uses material or immaterial human input, if the input is received before performance of the process or operation. Human input is deemed to be material if such input influences how the process or operation will be performed. Human input that consents to the performance of the process or operation is not deemed to be "material".

The term "computer-readable medium" as used herein refers to any tangible storage and/ or transmission medium that participate in providing instructions to a processor for execution. Such a medium may take many forms, including but not limited to, non-volatile media, volatile media, and transmission media. Non-volatile media includes, for example, NVRAM, or magnetic or optical disks. Volatile media includes dynamic memory, such as main memory. Common forms of computer-readable media include, for example, a floppy disk, a flexible disk, hard disk, magnetic tape, or any other magnetic medium, magneto-optical medium, a CD-ROM, any other optical medium, punch cards, paper tape, any other physical medium with patterns of holes, a RAM, a PROM, and EPROM, a FLASH-EPROM, a solid state medium like a memory card, any other memory chip or cartridge, a carrier wave as described hereinafter, or any other medium from which a computer can read.

A digital file attachment to e-mail or other self-contained information archive or set of archives is considered a distribution medium equivalent to a tangible storage medium. When the computer-readable media is configured as a database, it is to be understood that the database may be any type of database, such as relational, hierarchical, object-oriented, and/ or the like. Accordingly, embodiments may include a tangible storage medium or distribution medium and prior art-recognized equivalents and successor media, in which the software embodiments of the presently claimed invention are stored.

The terms "determine", "calculate" and "compute," and variations thereof, as used herein, are used interchangeably and include any type of methodology, process, mathematical operation or technique.

A module that performs a function also may be referred to as being configured to perform the function, e.g., a data module that receives data also may be described as being configured to receive data. Configuration to perform a function may include, for example: providing and executing computer code in a processor that performs the function; providing provisionable configuration parameters that control, limit, enable or disable capabilities of the module (e.g., setting a flag, setting permissions, setting threshold levels used at decision points, etc.); providing a physical connection, such as a jumper to select an option, or to enable / disable an option; attaching a physical communication link; enabling a wireless communication link; providing electrical circuitry that is designed to perform the function without use of a processor, such as by use of discrete components and/ or non-CPU integrated circuits; energizing a circuit that performs the function (e.g., providing power to a transceiver circuit in order to receive data); and so forth. Also, while the presently claimed invention is described in terms of exemplary embodiments, it should be appreciated those individual aspects of the presently claimed invention can be separately claimed.

The terms "healthy" and "healthier" and variations thereof, as used herein, are used interchangeably and refer to the enjoyment of improved vigor of an individual's mind, body and spirit.

The presently claimed invention, in an embodiment, may be used in association with health insurance companies and large employers to motivate their members or employees via the use of incentives to make healthier buying decisions when eating at restaurants. The presently claimed invention, in an embodiment, may be used in association with restaurants that want to promote healthier choices and gain insights into consumer preferences. The presently claimed invention can load restaurant menus into the presently claimed system and a client application, and assign client application reward points to meals based on their nutrient density. The client application may record customer food choices and credit them with the reward points based on the nutrient density of their meal. The presently claimed invention motivates consumers to make healthier buying decisions at restaurants by rewarding them with reward points based on the nutritional density of their meal choices and, in an embodiment, may provide a targeted messaging alert to customers about new healthier options on the restaurant menu. Earned reward points would be redeemable for free meals, healthy products at the supermarket, gym memberships, entertainment, health insurance discounts and other valued rewards.

There are many benefits attributable to the presently claimed invention. Large companies that insure their employees would pay for their members/ employees to participate in a program to motivate an individual to make healthier purchase decisions with the goal of a reduction in overall healthcare costs. In turn, health insurance companies would pay for their members to participate in the program with the similar goal of a reduction in healthcare costs. Participating members would earn redeemable points for healthier eating decisions. Organic grocers and growers would also benefit by an increased market demand and customer base. Restaurants would have the ability to earn customer loyalty, obtain better purchasing data and the ability to influence buying behavior. Healthier purchasing decisions would in turn potentially benefit health insurance companies and large employers by lowering costs, and providing the ability to better understand and influence customer behavior. Food brands, food associations, grocers and supermarkets may utilize reward points to attract, promote and motivate new customers to try and ultimately purchase their products.

In one embodiment of the presently claimed invention, there is provided a system and method whereby a healthcare insurance provider may customize reward points and reward point values based on dietary needs of the healthy choice member consumer. For example, if a member consumer is diagnosed as pre-diabetic or Type -1 or Type-2 diabetic, then choosing foods with low glycemic indexes will gain greater reward points per food item or drink than other types of healthy, albeit high glycemic index, foods and drinks.

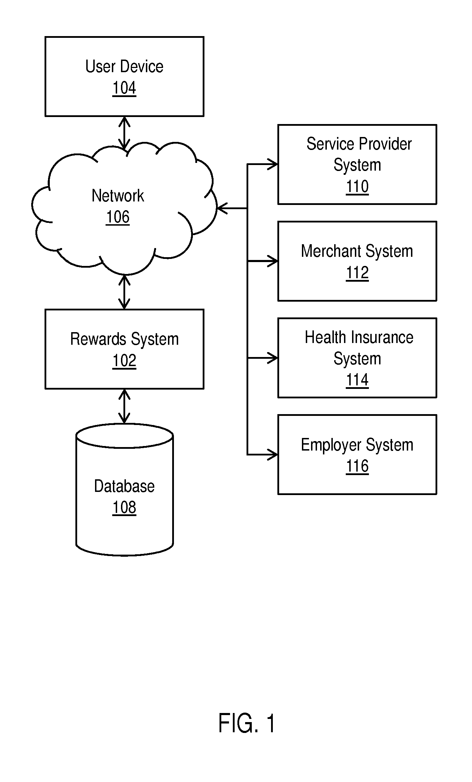

FIG. 1 illustrates a rewards system 102 for executing a rewards and loyalty program communicatively coupled to a user device 104 via a network 106 and a database 108, according to an embodiment of the presently claimed invention. The rewards system 102 can communicate with a service provider system 110, a merchant system 112, a health insurance system 114, and an employer system 116 via the network 106.

The rewards system 102 of the presently claimed invention may comprise a server for executing one or more functions to carry out methods of the presently claimed invention. The rewards system 102 may receive a food order from the user device 104. The food order may be received via the network 106 which communicatively couples the user device 104 and the rewards system 102. In one embodiment, the user device 104 may include but not be limited to a mobile phone, a personal digital assistant (PDA), a tablet, a laptop, a palmtop, a desktop, a personal computer, a phablet, or any other similar handheld device. In one embodiment, the network 106 may be any network including, but not limited to, a Local Area Network (LAN), Wide Area Network (WAN), SCSI, ATA, and the Internet or a combination thereof.

The rewards system 102 on receiving the food order may analyze the food order. In one embodiment, the analysis may include determining food or energy density of one or more food items in the food order. Based on the determined food density the rewards system 102 may present to the user at the user device 104 one or more recommendations regarding the food order. In one embodiment, the one or more recommendations may include presenting to the user a healthy food item option along with a number of reward points if the user accepts the recommendation. In another embodiment, the one or more recommendation may include presenting to the user a number of reward points if the user skips a food item in the food order. When the user accepts one or more recommendations of the rewards system 102, an account associated with the user may be updated with reward points corresponding to the accepted one or more recommendations.

The rewards system 102, in an embodiment, communicates with a service provider system 110. The service provider system 110 may include but is not limited to a restaurant, a hotel, an online food ordering system, a food delivery service, a coffee shop, a confectionary store etc. The service provider system 110 may provide a discount to the user for choosing healthy food items in the food order. The rewards system 102 may further communicate with a health insurance system 114 for receiving health information of the user. The one or more recommendations may be based on the health information of the user. Further, the rewards system 102 may provide information relating to user acceptance or decline of the one or more recommendations to the health insurance system 114. If the user accepts the recommendations, the health insurance system 114 may provide a benefit to the user in the form of a discounted insurance premium or similar cost savings benefit. If the user does not accept the recommendations no additional benefits would be provided. In one embodiment, based on user preferences and criteria established by the health insurance system 114, if the user declines more than a threshold number of recommendations for healthy food items by the rewards system 102, a penalty may be imposed on the user, such as an increase in the user's health insurance premium. In another embodiment, in lieu of a penalty on the user, the rewards system 102 may not make available unhealthy food items for the user to select. In other words, the rewards system 102 may modify a food menu by deleting unhealthy food items for the user, and presenting the user only the modified food menu from which to compose the food order.

The rewards system 102 may be communicatively coupled to the employer system 116. The employment system 116 includes employment information of the user. The employment information may include health insurance policy provided by an employer to the user in accordance with human resource policies of the employer. The employer system 116 may define criteria for the rewards system 102 for incentivizing the user when the user is an employee associated with the employer system. The database 108 may be communicatively coupled to the rewards system 102. The rewards system 102 may store data or information associated with the user, the user device 104, the service provider system 110, the merchant system 112, the health insurance system 114 and/ or, the employer system 116. The database 108 may be external to the rewards system 102. In one embodiment, the database 108 may be implemented inside the rewards system 102 in form of a computer readable medium. In one embodiment, the computer readable medium is tangible and non-transitory. The database 108 may be implemented as a combination of one or more storage devices.

Further, the rewards system 102 is communicatively coupled to the merchant system 112 via the network 106. The merchant system 112 provides platform to the user for redeeming reward points earned by the user. The merchant system 112 may provide the user one or more options for redeeming the reward points. The one or more options may include but not be limited to restaurants and entertainment, food and beverage, apparel and accessories, home and garden, books and magazine, digital downloads, airline miles etc. In one embodiment the digital downloads may include music, video, books, apps, and games etc. The rewards system 102 may update the user account of reward points based on the user redeeming the reward points at the platform offered by the merchant system 112.

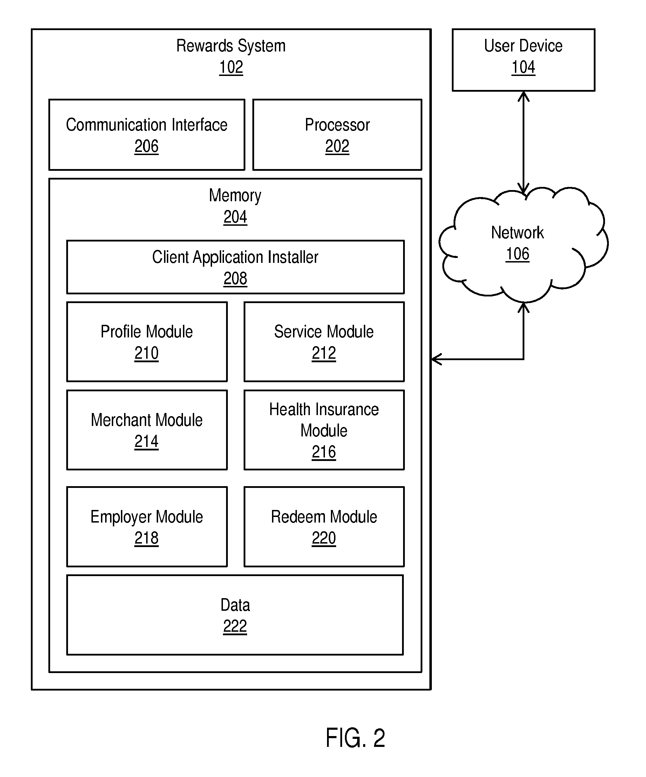

FIG. 2 illustrates the architecture of the rewards system, according to an embodiment of the presently claimed invention. The rewards system 102 can communicate with the user device 104 via the network 106. The rewards system includes a processor 202, a memory 204, a communication interface 206, a client application installer 208, and one or more modules. In one embodiment, the one or more modules are stored in the memory 204, which can be accessed by the processor 202. The processor 202 may communicate with the memory 204, the communication interface 206, the client application installer 208 and the one or more modules to carry out one or more functions of the rewards system 102. For example, the processor 202 may read data and computer readable instructions from the memory 204 for providing healthy food recommendations to the user. The communication interface 206 facilitates communications with user device 104 or any other outside device or system via the network 106. The client application installer 208 facilitates the installation of a client application at the user device 104. The client application may be used by the user for carrying out the food order transactions with the rewards system 102. The client application may be downloaded to the user device 104 via online platforms other than the rewards system 102.

The one or more modules include a profile module 210, a service module 212, a merchant module 214, a health insurance module 216, an employer module 218, and a redeem module 220.

A module of the one or more modules may include a program code which when executed by the processor 202 carries out one or more functions associated with the module. The program code may be a machine readable code stored in the memory 204. In one embodiment, the program code may be stored in a storage device different from the memory 204 and fetched to the memory 204 for execution by the processor. The module may be a logical combination of computer readable instructions when executed by the processor 202 performs a task or function associated with the module. In one embodiment, the program code may be implemented in hardware. In this embodiment, the module may take form of a self-sufficient machine which when powered performs a task or function associated with the module. One or more such hardware modules may be combined to perform one or more tasks to be performed by the rewards system 102. In the case where the one or modules are implemented in hardware, the processor 202 can be relieved of one or more tasks performed by the one or more modules. In one embodiment, a module can be partially implemented in hardware and partially in software. Simple and repetitive task can be implemented in hardware for faster execution and more complex logical tasks may be implemented in software. The hardware and software portions of the module may coordinate with another to achieve the overall functionality of the module. The profile module 210, the service module 212, the merchant module 214, the health insurance module 216, the employer module 218, and the redeem module 220 described in detail below may be implemented in hardware or software or a combination thereof as discussed hereinabove.

The profile module 210 creates a profile of the user based on inputs received via the user device 104. The information associated with the profile may be stored as profile data in the memory 204. In one embodiment, the profile data may be stored in an external database such as the database 108. The profile data may include name, address, contact information, social security number, demographic information or any other information associated with the user. The profile data may be accessed by the user via a user name and password combination or any other user authentication mechanism. The profile data may also be accessed by the service module 212 for providing healthy food recommendation to the user. The profile data may further include one or more medical conditions of the user.

The profile module 210 may further store user preference data in the memory 204. The preference data may be accessed by the processor 202 and the one or more modules. The preference data may include but not limited to one or more preferences of the user related to type of food, favorite restaurants, preferred maximum distance of the restaurants from home location or current location of the user.

The service module 212 may provide one or more services to the user of the rewards system 102. Data or information associated with the one or more services may be stored as service data in the memory 204. The service data, in an embodiment, may include, but not limited to, data and information associated with transactions carried out by the user at the rewards system. The transactions may include ordering food via the rewards system 102. The service data may include the recommendations provided to the user, recent transactions of the user, reward points earned by the user for each transaction etc. The service data may further include one or more menus of one or more restaurants.

The merchant module 214, in an embodiment, may register one or more merchants with rewards system 102. The merchant module 214 may store data or information associated with one or more merchants. The health insurance module 216, in an embodiment, may retrieve health insurance data from the health insurance system 114. The health insurance data may be used to define one or more criterion for providing rewards points or discount to the user.

The employer module 218, in an embodiment, may be configured to instruct the processor 202 to retrieve employer data from the employer system 116. The employer data may be used to define one or more criterion for providing rewards points or discount to the user. The redeem module 220 to facilitate redeeming of reward points by the user at the one or more merchants.

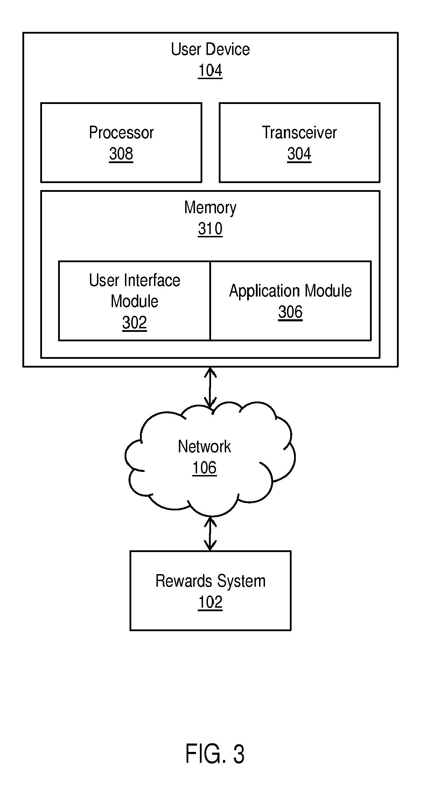

FIG. 3 illustrates the architecture of the user device 104, according to an embodiment of the presently claimed invention. The user device 104 can communicate with the rewards system 102 via network 106. The user device 104 includes a transceiver 304, a processor 308, and a memory 310 that includes a user interface module 302 and an application module 306. The user interface module 302 may generate and present a user interface at the user device 104. The user interface, in an embodiment, is used by the user for ordering food at the rewards system 102, getting recommendations for healthy food items, accepting or rejecting the recommendations, creating a user profile, and redeeming of reward points. Data or information associated with user interactions between the user device 104 and the reward points 102 may be stored by the processor 308 in the memory 310. In an embodiment, the memory 310 includes one or more computer readable instructions which may be read by the processor to perform one or more functions including but not limited to ordering food at the rewards system 102, getting recommendations for healthy food items, accepting or rejecting the recommendations, creating a user profile, and redeeming of reward points. The application module 306 may interact with the processor 308 to install the client application at the user device 104 based on communications with the client application installer 208 of the rewards system 102. The communication between the user device 104 and the rewards system 102 or any other device or system is via the transceiver 304.

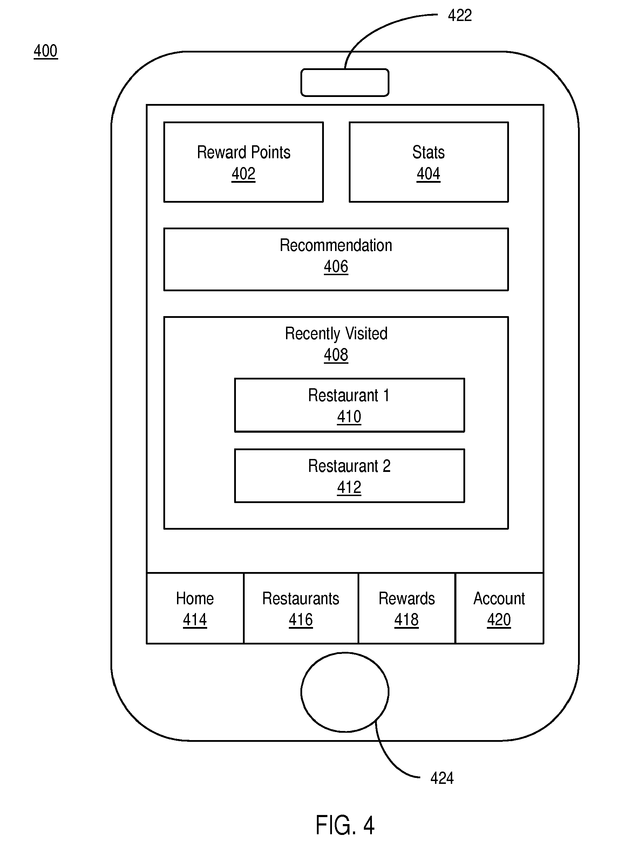

FIG. 4 illustrates an instance of a user interface 400 of the user device 104 depicting an initial interaction of the user with the rewards system 102. The user device 104 may include a microphone 422. The microphone 422 may be configured to receive audio instructions from the user to perform one or more functions of the presently claimed invention. For example, in one embodiment, the user may order the food via audio instructions to the user device 104. Reward points 402 represents total reward points associated with the user account of the user. Stats 404 may represent, in an embodiment, statistical data associated with the user account. The statistical data 404 may include but not limited to per meal reward points earned by the user. The statistical data may include reward points earned by the user in a previous time period. The previous order may be any one of a day, week, month, year or any other preconfigured time duration. In one embodiment, the statistical data may include reward points earned by other users. Recommendations 406 may represent one or more recommendations provided to the user. The recommendation may include positive recommendations and negative recommendations. The positive recommendations may include recommendation to the user for ordering a healthy food item. The negative recommendation may include recommendation to the user for skipping or not ordering an unhealthy food item. Each recommendation may carry corresponding reward points. Recently visited 408 may show on the user interface one or more restaurants, such as restaurant 1 410 and restaurant 2 412, recently visited by the user. Home 414 may represent a soft or hard button when pressed takes the user to a home screen. The restaurants 416 may represent a soft or hard button to access information relating to one or more restaurants. The rewards 418 may represent a soft or hard button to access complete information related to reward points associated with user. The account 420 may represent a soft or hard button to access account information associated with the user. The account information may include profile information and user preference of the user. The user device may further include a hard or soft button 424 which may reset the user device 102 and exit the user interface of the client application.

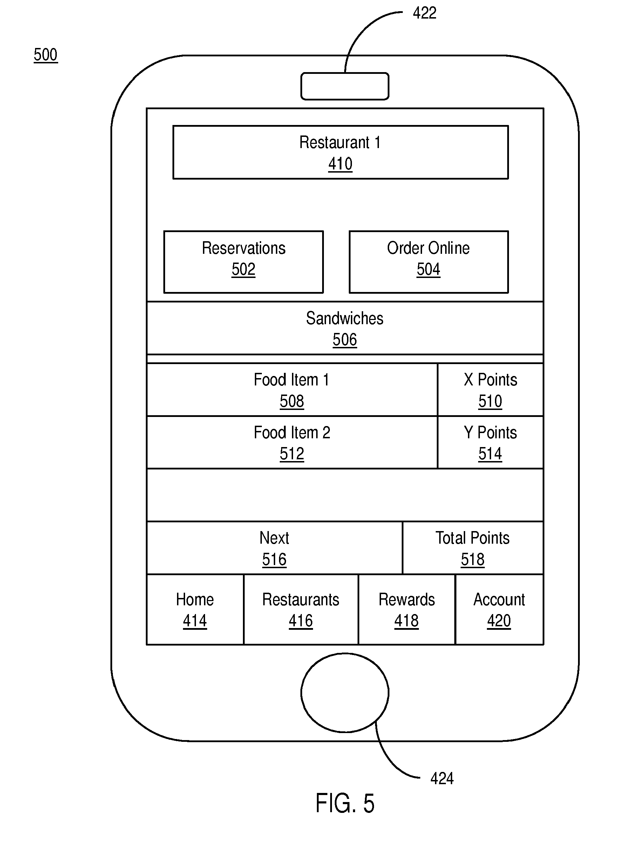

FIG. 5 illustrates an instance of a user interface of the user device 104 depicting rewards points associated with a recommendation of a healthy food item by the rewards system 102. Restaurant 1 410 represents the restaurant from which the user is currently ordering. The user can make a reservation at the restaurant 1 410 using the soft or hard button reservations 502. The user can make an order online at the restaurant 1 410 using the soft or hard button order online 504. Sandwiches 506 may represent a button to access further information on sandwiches available at the restaurant 1 410. The user interface may further show to the user food item 1 508 and food item 2 516 and corresponding reward points, X points 510 and Y points 514. Total rewards points are shown by total points 518. A next button 516 may take the user to a next screen.

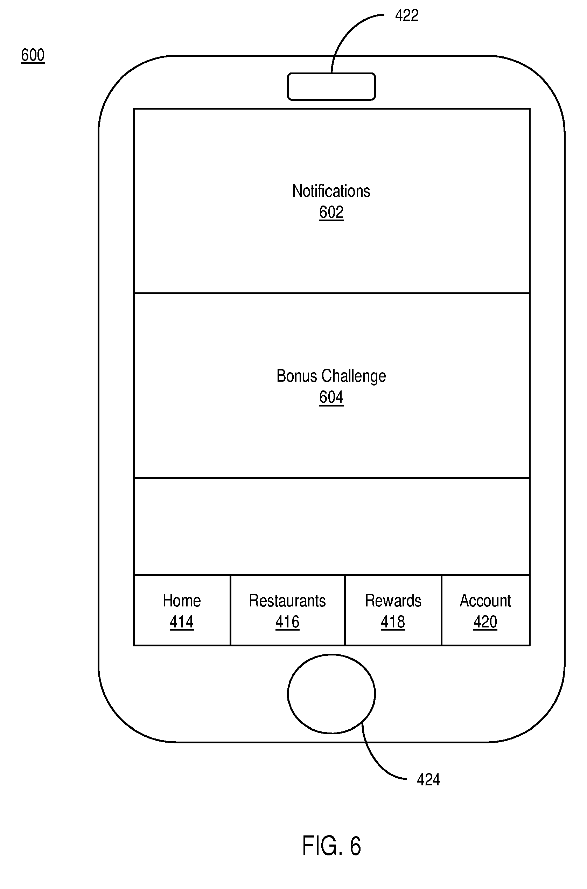

FIG. 6 illustrates an instance of a user interface 600 of the user device 104, according an embodiment of the presently claimed invention. Notifications 602 show one or more notifications to the user. The notifications 602 may include notifications or messages related to availability of one or more new food items available at a restaurant. The notifications 602 may also include a change in criteria for awarding reward points. The notifications 602 may further include information regarding expiry of reward points. The notifications 602 may also include information relating to the users. For example, the user may be notified when a family member or friend of the user joins the rewards system 102. The notifications 602 may further include any other information associated with the user account. The bonus challenge 604 may be a negative recommendation for skipping a healthy food item and associated bonus reward points. In one embodiment, the bonus reward points may be provided to the user if the user accepts a threshold number of recommendations for the healthy food items.

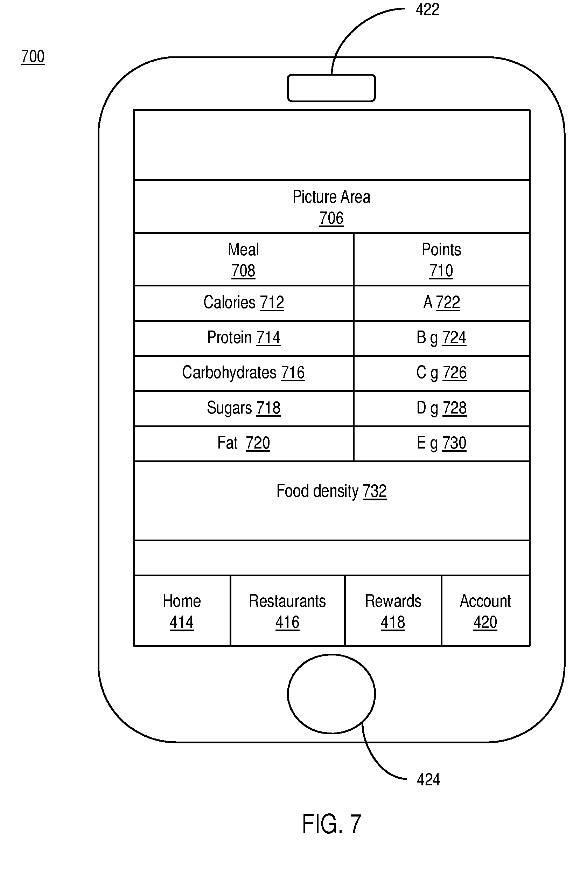

FIG. 7 illustrates an instance of a user interface 700 of the user device 104, according an embodiment of the presently claimed invention. A picture area 706 may be used by user to include a photograph of the user or any other photograph. If the user does not upload a photograph in the picture area 706, a default picture may be shown there. The user interface 700 further shows an ordered meal 708 along with reward points, points 710. The user interface 700 further shows a food density 732 for the meal 708. The food density 732 may be determined based at least on one or more of A 722 (number of calories) shown against calories 712; B 714 grams of protein shown against protein 714; C 726 grams of carbohydrates shown against carbohydrates 716; D 218 grams of sugars shown against sugars 718; E 730 grams of fats shown against fat 720. Any other unit instead of calories and grams for showing nutrient information of the meal 708 may be used. A threshold food density may be preconfigured in the rewards system 104. In one embodiment, the recommendation may be provided to the user only when the food density 732 is greater than or equal to the preconfigured food density.

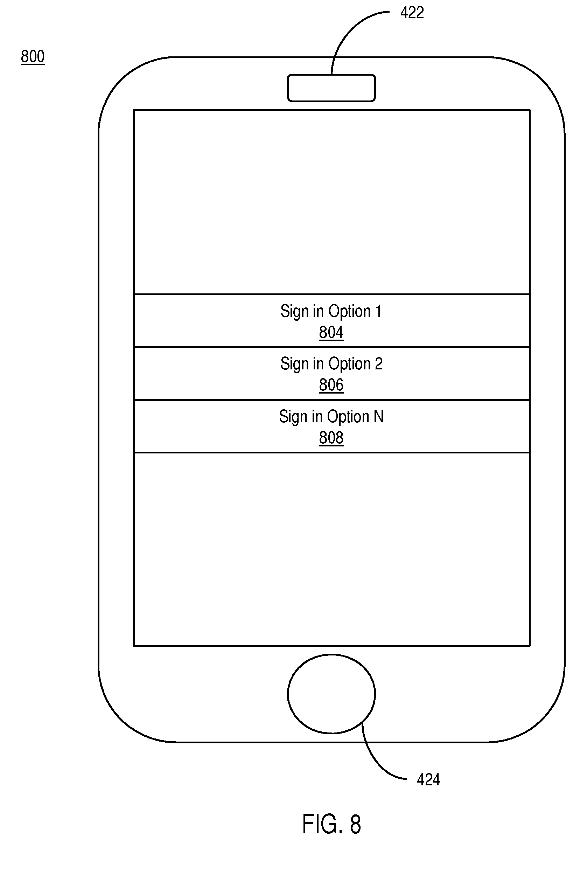

FIG. 8 illustrates an instance of a user interface 800 of the user device 104, according an embodiment of the presently claimed invention. The user interface 800 may be used by the user to login into the client application using any of login options sign in Option 1 804, sign in Option 2 806, or sign in Option N. The login options may be proprietary to the rewards system 102. In one embodiment, the login options may be login via a social media platform. In one embodiment, the login options may be login via an email system.

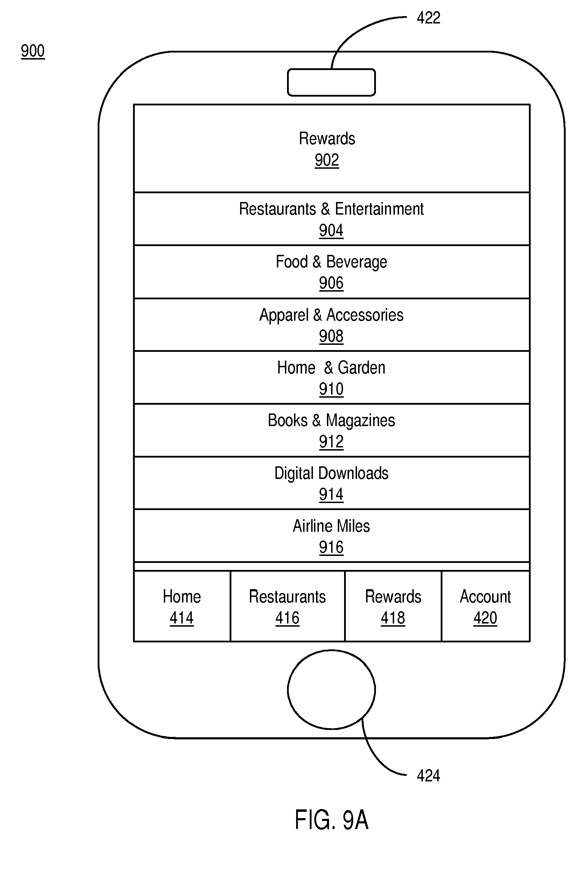

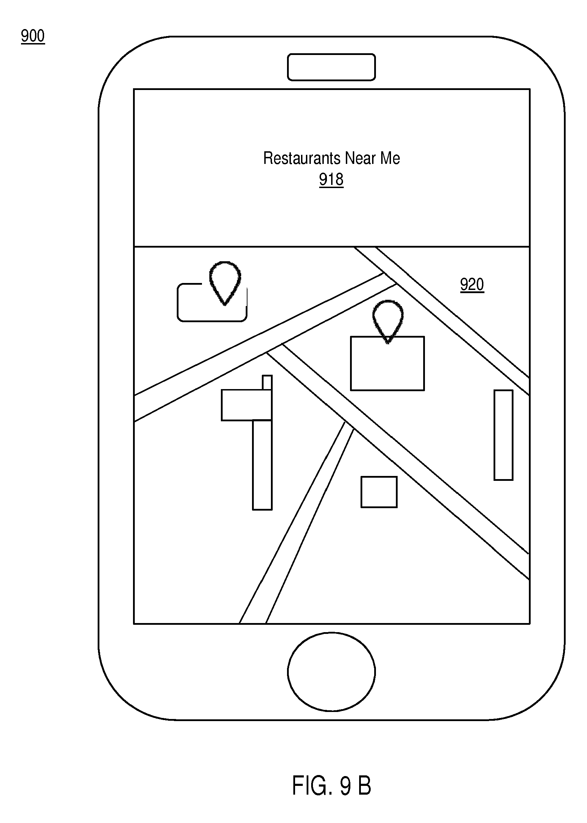

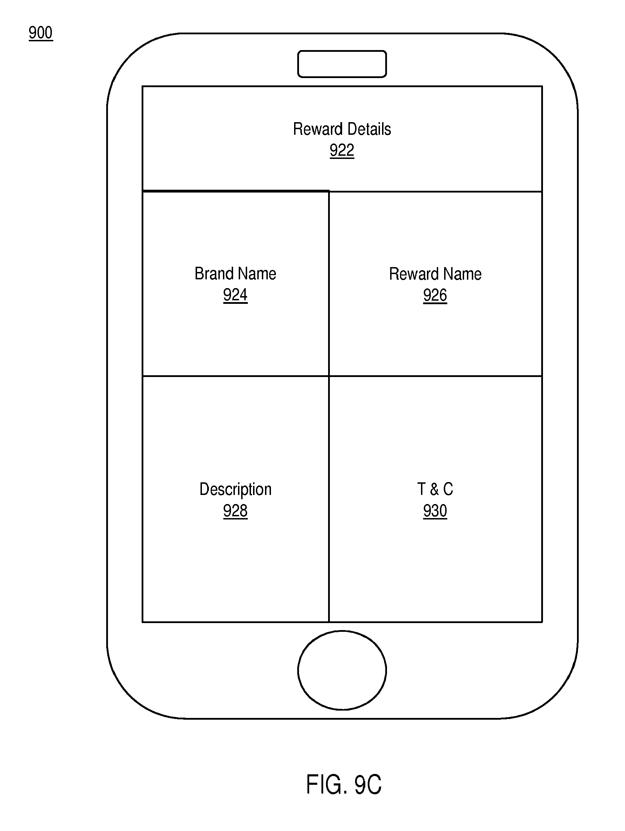

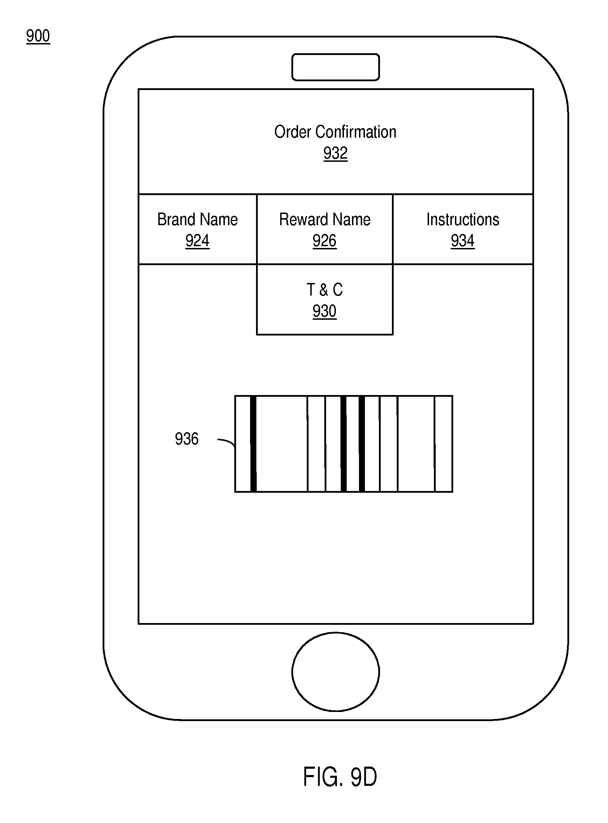

FIG. 9A illustrates an instance of a user interface 900 of the user device 104, according an embodiment of the presently claimed invention. The user may be shown rewards 902 and one or more options to redeem the reward points. The one or more options may include, but not limited to purchasing one or more products or services associated with Restaurants & Entertainment 904, Food & Beverage 906, Apparel & Accessories 908, Home & Garden 910, Books & Magazines 912, Digital Downloads 914, and Airline Miles 916. FIG 9B shows, in an embodiment, restaurants near a user by a restaurants near me 918 item. The user can search for the location of a restaurant using a map 920 which may be integrated or communicatively coupled to the client application. FIG. 9C illustrates rewards details 922, in an embodiment. A brand name 924, a reward name 926, a description 928, and terms and conditions, T & C 930, associated with the reward may be shown to the user. FIG. 9D illustrates, in an embodiment, an order confirmation 932 screen. The order confirmation 932 screen may show to the user the brand name 924, the reward name 926, the T & C 930 and instructions 934. The instructions 934 may include instructions to the user on delivery mode, timeline of delivery of the reward, and/ or a collection point for collecting the reward. The order confirmation 932 screen further shows a bar code 936 associated with the reward.

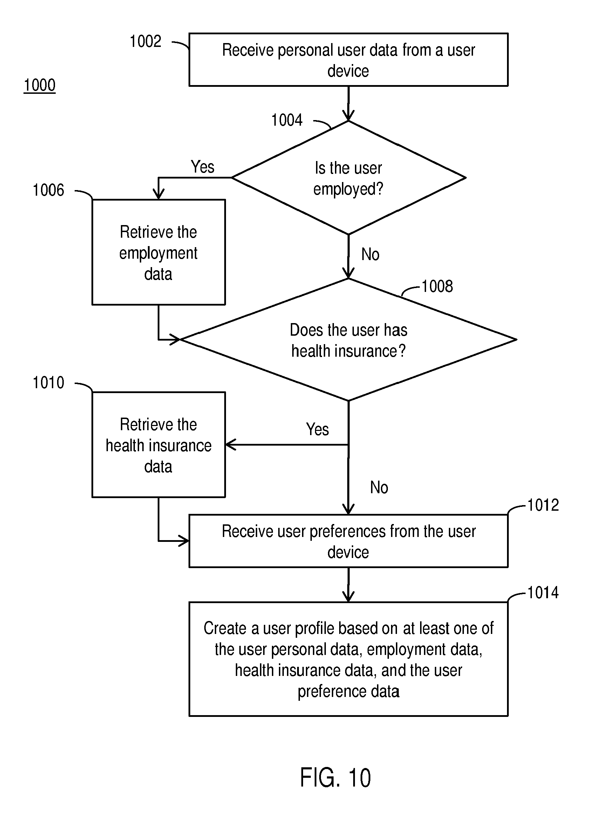

FIG. 10 illustrates an exemplary method 1000 for creating a user profile at the rewards system, according to an embodiment of the presently claimed invention. The method 1000 may begin at step 1002. At step 1002, the rewards system 102 may receive personal user data from the user device 104. At step 1004, it is determined whether the user employed. When the user is employed, at step 1006, the rewards system 102 may retrieve the employment data of the user. When the user is not employed or after retrieving of the employment data the method 1000 proceeds to step 1008. At step 1008, it is determined whether the user has a health insurance. When the user has a health insurance, at step 1008, the rewards system 102 may retrieve the health insurance data of the user. When the user does not has a health insurance or after retrieving of the health insurance data the method 1000 proceeds to step 1012. At step 1012, the rewards system 102 may receive user preferences from the user device 104. At step 1014, the rewards system 1014 may create a user profile based on at least one of the user personal data, employment data, health insurance data, and the user preference data.

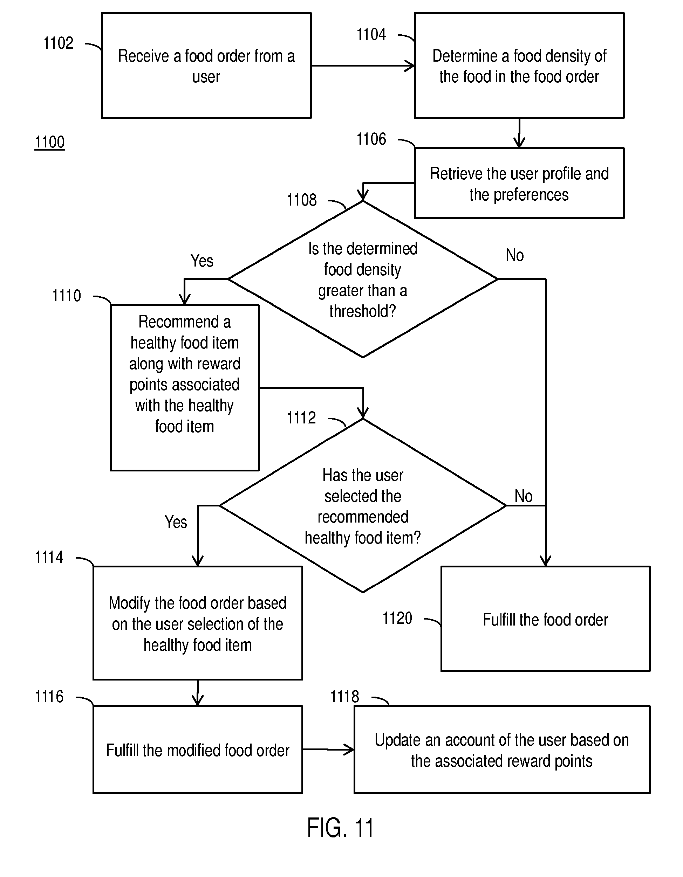

FIG. 11 illustrates an exemplary method 1100 for fulfilling a food order at the rewards system 102, according to an embodiment of the presently claimed invention. The method 1100 may begin at step 1102. At step 1102, a food order is received from a user. At step 1104 a food density of the food in the food order is determined. At step 1106, a user profile and user preferences are retrieved. At step 1108, it is determined whether the determined food density is greater than or equal to a preconfigured food density threshold. When the food density is less than the threshold, the food order is fulfilled at step 1120. When the food density is greater than or equals to the threshold the method 1100 proceeds to step 1110. At step 1110, the rewards system 102 recommends a healthy food item along with reward points associated with the healthy food item. At step 1112, it is determined whether the user has selected the recommended healthy food item. When the user has not selected the recommended healthy food item, the food order is fulfilled at step 1120. When the user has selected the recommended healthy food item, the method 1100 proceeds to step 1114. At step 1114, the rewards system 102 may modify the food order based on the user selection of the healthy food item. At step 1116, the modified food order is fulfilled by the rewards system 102. At step 1118, the rewards system 102 may update an account of the user based on the associated reward points.

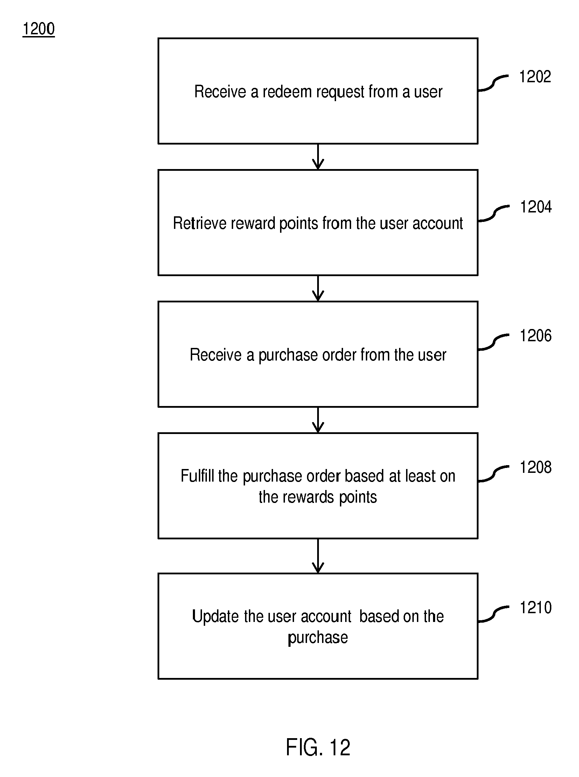

FIG. 12 illustrates an exemplary method 1200 for redeeming reward points by the user, according to an embodiment of the presently claimed invention. The method 1200 may begin at step 1202. At step 1202 a redeem request from a user may be received at the rewards system 102. At step 1204, the reward points from the user account may be retrieved. At step 1206, a purchase order from the user is received. At step 1208, the purchase order based at least on the rewards points is fulfilled by the rewards system 102 in coordination with the merchant system 112. At step 1210, the user account may be updated by the rewards system 102 based on the purchase order of the user.

Thus, as per various embodiment of the presently claimed invention, a computer implemented method for executing a rewards and loyalty program is disclosed. The method includes instructing a processor communicatively coupled to computer readable memory comprising computer readable instructions, to receive a food order from a user, determine a food density of food items in the food order, retrieve a user profile and user preferences associated with the user, recommend a healthy food item along with reward points associated with the healthy food item based on the user profile and the user preferences when the determined food density is greater than or equal to a threshold. The method may further include a step of receiving a selection of the recommended healthy food item from the user. The method may further include a step of modifying the food order based on the user selection of the recommended healthy food item. The method may further include a step of fulfilling the modified food order. The method may further include a step of updating an account of the user with reward points based on user selection of the recommended healthy food items. The food order may be received via a graphical user interface of a user device communicatively coupled to a rewards system with a communication network. The step of recommending of the healthy food item is further based at least on a time weighted average of previous food orders of the user. The rewards points may be based on at least one of employer data, health insurance data, and service provider data associated with the user. The method may further include step of redeeming reward points by the user using a merchant system communicatively coupled to a rewards system. The merchant system comprises at least one of a product and a service associated with a restaurant, an apparels store, a book store, a digital download store, and an airline.

Further, as per various embodiment of the presently claimed invention a rewards system for executing a rewards and loyalty program. The system includes a computer readable memory comprising computer readable instructions; the memory communicatively coupled to a processor, the processor is configured to execute the computer readable instructions. The system includes a service module configured to instruct the processor to receive a food order from a user, determine a food density of food items in the food order, and recommend a healthy food item along with reward points associated with the healthy food item based on the user profile and the user preferences when the determined food density is greater than or equal to a threshold. The system may further include a profile module configured to instruct the processor to retrieve a user profile and user preferences associated with the user.

Furthermore, while the exemplary embodiments of the presently claimed invention illustrated herein show the various components of the system collocated, certain components of the system can be located remotely, at distant portions of a distributed network, such as a LAN and/ or the Internet, or within a dedicated system. Thus, it should be appreciated, that the components of the system can be combined in to one or more devices, such as a switch, server, and/ or adjunct, or collocated on a particular node of a distributed network, such as an analog and/ or digital telecommunications network, a packet-switch network, or a circuit-switched network.

It will be appreciated from the preceding description, and for reasons of computational efficiency, the components of the system can be arranged at any location within a distributed network of components without affecting the operation of the system. For example, the various components can be located in a switch such as a PBX and media server, gateway, in one or more communications devices, at one or more users' premises, or some combination thereof. Similarly, one or more functional portions of the system could be distributed between a telecommunications device(s) and an associated computing device.

Furthermore, it should be appreciated the various links connecting the elements can be wired or wireless links, or any combination thereof, or any other known or later developed element(s) that is capable of supplying and/ or communicating data to and from the connected elements. These wired or wireless links can also be secure links and may be capable of communicating encrypted information. Transmission media used as links, for example, can be any suitable carrier for electrical signals, including coaxial cables, copper wire and fiber optics, and may take the form of acoustic or light waves, such as those generated during radio-wave and infra-red data communications.

Also, while the flowcharts have been discussed and illustrated in relation to a particular sequence of events, changes, additions, and omissions to this sequence can occur without materially affecting the operation of embodiments of the present invention.

A number of variations and modifications of the presently claimed invention can be used. It would be possible to provide for some features of the presently claimed invention without providing others.

For example in one alternative embodiment of the presently claimed invention, the systems and methods of this presently claimed invention can be implemented in conjunction with a special purpose computer, a programmed microprocessor or microcontroller and peripheral integrated circuit element(s), an ASIC or other integrated circuit, a digital signal processor, a hard-wired electronic or logic circuit such as discrete element circuit, a programmable logic device or gate array such as PLD, PLA, FPGA, PAL, special purpose computer, any comparable means, or the like.

In general, any device(s) or means capable of implementing the methodology illustrated herein can be used to implement the various aspects of this presently claimed invention. Exemplary hardware that can be used for the presently claimed invention includes computers, handheld devices, telephones (*e.g*., cellular, Internet enabled, digital, analog, hybrids, and others), and other hardware known in the art. Some of these devices include processors (*e.g*., a single or multiple microprocessors), memory, non-volatile storage, input devices, and output devices. Furthermore, alternative software implementations including, but not limited to, distributed processing or component/ object distributed processing, parallel processing, or virtual machine processing can also be constructed to implement the methods described herein.

In yet another embodiment of the presently claimed invention, the disclosed methods may be readily implemented in conjunction with software using object or object-oriented software development environments that provide portable source code that can be used on a variety of computer or workstation platforms. Alternatively, the disclosed system may be implemented partially or fully in hardware using standard logic circuits or VLSI design. Whether software or hardware is used to implement the systems in accordance with embodiments of the presently claimed invention is dependent on the speed and/ or efficiency requirements of the system, the particular function, and the particular software or hardware systems or microprocessor or microcomputer systems being utilized.

In yet another embodiment of the presently claimed invention, the disclosed methods may be partially implemented in software that can be stored on a storage medium, executed on programmed general-purpose computer with the cooperation of a controller and memory, a special purpose computer, a microprocessor, or the like. In these instances, the systems and methods of this present invention can be implemented as program embedded on personal computer such as an apple, JAVA® or CGI script, as a resource residing on a server or computer workstation, as a routine embedded in a dedicated measurement system, system component, or the like. The system can also be implemented by physically incorporating the system and/ or method into a software and/ or hardware system.

Although the presently claimed invention describes components and functions implemented in the embodiments with reference to particular standards and protocols, it is not limited to such standards and protocols. Other similar standards and protocols not mentioned herein are in existence and considered to be included in the present invention. Moreover, the standards and protocols mentioned herein and other similar standards and protocols not mentioned herein are periodically superseded by faster or more effective equivalents having essentially the same functions. Such replacement standards and protocols having the same functions are considered equivalents included in the presently claimed invention.

The presently claimed invention, in various embodiments, configurations, and aspects, includes components, methods, processes, systems and/ or apparatus substantially as depicted and described herein, including various embodiments, subcombinations, and subsets thereof. Those of skill in the art will understand how to make and use the presently claimed invention after understanding the present disclosure. The presently claimed invention, in various embodiments, configurations, and aspects, includes providing devices and processes in the absence of items not depicted and/ or described herein or in various embodiments, configurations, or aspects hereof, including in the absence of such items as may have been used in previous devices or processes, *e.g.,* for improving performance, achieving ease and/ or reducing cost of implementation.

The foregoing discussion of the presently claimed invention has been presented for purposes of illustration and description. It is not intended to limit the presently claimed invention to the form or forms disclosed herein. In the foregoing Detailed Description, for example, various features of the presently claimed invention are grouped together in one or more embodiments, configurations, or aspects for the purpose of streamlining the disclosure. The features of the embodiments, configurations, or aspects may be combined in alternate embodiments, configurations, or aspects other than those discussed above. This method of disclosure is not to be interpreted as reflecting an intention the presently claimed invention requires more features than are expressly recited in each claim. Rather, as the following claims reflect, inventive aspects lie in less than all features of a single foregoing disclosed embodiment, configuration, or aspect. Thus, the following claims are hereby incorporated into this Detailed Description, with each claim standing on its own as a separate embodiment of the presently claimed invention.

Moreover, though the description of the presently claimed invention has included description of one or more embodiments, configurations, or aspects and certain variations and modifications, other variations, combinations, and modifications are within the scope of the presently claimed invention, *e.g*., as may be within the skill and knowledge of those in the art, after understanding the present disclosure. It is intended to obtain rights which include alternative embodiments, configurations, or aspects to the extent permitted, including alternate, interchangeable and/ or equivalent structures, functions, ranges or steps to those claimed, whether or not such alternate, interchangeable and/ or equivalent structures, functions, ranges or steps are disclosed herein, and without intending to publicly dedicate any patentable subject matter

Furthermore, one or more of the following numbered clauses may describe and relate to further aspects or features within the context of the present teaching:.
1. A computer implemented method for executing a rewards and loyalty program, the method comprising:
   instructing a processor, communicatively coupled to a computer readable memory, the memory comprising computer readable instructions, to:
      receive a food order from a user;
      determine a food density of food items in the food order;
      retrieve a user profile and user preferences associated with the user; and
      recommend a healthy food item along with reward points associated with the healthy food item based on the user profile and the user preferences when the determined food density is greater than or equal to a threshold.
2. The method of clause 1, further comprising receiving a selection of the recommended healthy food item from the user.
3. The method of clause 2, further comprising modifying the food order based on the user selection of the recommended healthy food item.
4. The method of clause 3, further comprising fulfilling the modified food order.
5. The method of clause 4, further comprising updating an account of the user with reward points based on the user selection of the recommended healthy food items.
6. The method of clause 1, wherein the food order is received via a graphical user interface of a user device communicatively coupled to a rewards system with a communication network.
7. The method of clause 1, wherein the recommending of the healthy food item is further based at least on a time weighted average of previous food orders of the user.
8. The method of clause 1, wherein the rewards points are based on at least one of employer data, health insurance data, and service provider data associated with the user.
9. The method of clause 1, further comprising redeeming reward points by the user using a merchant system communicatively coupled to a rewards system.
10. The method of clause 9, wherein the merchant system comprises at least one of a product and a service associated with a restaurant, an apparels store, a book store, a digital download store, and an airline.
11. A rewards system for executing a rewards and loyalty program, the system comprising:
   a computer readable memory comprising computer readable instructions, the memory communicatively coupled to a processor, the processor configured to execute the computer readable instructions;
   a service module configured to instruct the processor to:
      receive a food order from a user; and
      determine a food density of food items in the food order;
   a profile module configured to instruct the processor to retrieve a user profile and user preferences associated with the user; and
   the service module further configured to instruct the processor to recommend a healthy food item along with reward points associated with the healthy food item based on the user profile and the user preferences when the determined food density is greater than or equal to a threshold.
12. The system of clause 11, wherein the service module is further configured to instruct the processor to receive a selection of the recommended healthy food item from the user.
13. The system of clause 12, wherein the service module is further configured to instruct the processor to modify the food order based on the user selection of the recommended healthy food item.
14. The system of clause 13, wherein the service module is further configured to instruct the processor to fulfill the modified food order.
15. The system of clause 14, wherein the service module is further configured to instruct the processor to update an account of the user with reward points based on user selection of the recommended healthy food items.
16. The system of clause 11, wherein the service module is further configured to instruct the processor to receive the food order via a graphical user interface of a user device communicatively coupled to a rewards system with a network.
17. The system of clause 11, wherein the service module is further configured to instruct the processor to recommend the healthy food item based at least on a time weighted average of previous food orders of the user.
18. The system of clause 11, wherein the rewards points are based on at least one of employer data, health insurance data, and service provider data associated with the user.
19. The system of clause 11, further comprising a redeem module configured to instruct the processor to process the redeeming of reward points by the user using a merchant system communicatively coupled to a rewards system.
20. The method of clause 19, wherein the merchant system comprises at least one of a product and a service associated with a restaurant, an apparels store, a book store, a digital download store, and an airline.

## Claims

1. A computer implemented method for executing a rewards and loyalty program, the method comprising:
instructing a processor, communicatively coupled to a computer readable memory, the memory comprising computer readable instructions, to:
receive a food order from a user;
determine a food density of food items in the food order;
retrieve a user profile and user preferences associated with the user; and
recommend a healthy food item along with reward points associated with the healthy food item based on the user profile and the user preferences when the determined food density is greater than or equal to a threshold.

2. The method of claim 1, further comprising receiving a selection of the recommended healthy food item from the user.

3. The method of claim 2, further comprising modifying the food order based on the user selection of the recommended healthy food item.

4. The method of claim 3, further comprising fulfilling the modified food order.

5. The method of claim 4, further comprising updating an account of the user with reward points based on the user selection of the recommended healthy food items.

6. The method of any preceding claim, being configured in at least one of the following ways:
wherein the food order is received via a graphical user interface of a user device communicatively coupled to a rewards system with a communication network;
wherein the recommending of the healthy food item is further based at least on a time weighted average of previous food orders of the user; and
wherein the rewards points are based on at least one of employer data, health insurance data, and service provider data associated with the user.

7. The method of any preceding claim, further comprising redeeming reward points by the user using a merchant system communicatively coupled to a rewards system, wherein the merchant system optionally comprises at least one of a product and a service associated with a restaurant, an apparels store, a book store, a digital download store, and an airline.

8. A rewards system for executing a rewards and loyalty program, the system comprising:
a computer readable memory comprising computer readable instructions, the memory communicatively coupled to a processor, the processor configured to execute the computer readable instructions;
a service module configured to instruct the processor to:
receive a food order from a user; and
determine a food density of food items in the food order;
a profile module configured to instruct the processor to retrieve a user profile and user preferences associated with the user; and
the service module further configured to instruct the processor to recommend a healthy food item along with reward points associated with the healthy food item based on the user profile and the user preferences when the determined food density is greater than or equal to a threshold.

9. The system of claim 8, wherein the service module is further configured to instruct the processor to receive a selection of the recommended healthy food item from the user.

10. The system of claim 9, wherein the service module is further configured to instruct the processor to modify the food order based on the user selection of the recommended healthy food item.

11. The system of claim 10, wherein the service module is further configured to instruct the processor to fulfill the modified food order.

12. The system of claim 11, wherein the service module is further configured to instruct the processor to update an account of the user with reward points based on user selection of the recommended healthy food items.

13. The system of any of claims 8 to 12, wherein the service module is further configured to instruct the processor to receive the food order via a graphical user interface of a user device communicatively coupled to a rewards system with a network.

14. The system of any of claims 8 to 13, being configured in at least one of the following ways:
wherein the service module is further configured to instruct the processor to recommend the healthy food item based at least on a time weighted average of previous food orders of the user; and
wherein the rewards points are based on at least one of employer data, health insurance data, and service provider data associated with the user.

15. The system of any of claims 8 to 14, further comprising a redeem module configured to instruct the processor to process the redeeming of reward points by the user using a merchant system communicatively coupled to a rewards system, wherein optionally the merchant system comprises at least one of a product and a service associated with a restaurant, an apparels store, a book store, a digital download store, and an airline.
